# EUROPEAN PATENT APPLICATION

(11) **EP 4 725 420 A1**
(43) Date of publication of application: **15.04.2026**
(21) Application number: 25214337.5
(22) Date of filing: 07.11.2025
(51) Int. Cl.: A61B 5/352, A61B 5/366, A61B 5/00

(54) **METHOD FOR AUTOMATIC ANALYSIS OF ELECTROCARDIOGRAPHIC, ECG, RECORDINGS, SYSTEM FOR AUTOMATIC ANALYSIS OF ELECTROCARDIOGRAPHIC, ECG, RECORDINGS, COMPUTER PROGRAM PRODUCT, AND COMPUTER-READABLE DATA CARRIER**

(71) Applicant: Cardiomatics Sp. z o.o., 31-339 Krakow (PL)
(72) Inventor: Barczewska, Katarzyna, Krakow (PL)
(74) Representative: AOMB Polska Sp. z.o.o.

(57) **Abstract**

The present invention relates to a method for automatic analysis of Holter recordings, which involves receiving an ECG recording containing multiple channels, filtering and resampling the data to a uniform frequency, and assessing signal quality for each channel to determine low-quality probabilities for short time segments. Channels or segments with signal quality below a predetermined threshold are excluded. Subsequently, R-peak candidates are detected and then verified and classified. The invention also relates to a system, a computer product and a computer-readable data carrier.

## Description

### TECHNICAL FIELD

The present invention relates to method, system, computer program product, and computer-readable data carrier for automatic analysis of electrocardiographic (ECG) recordings, in particular long-term Holter recordings. More specifically, it concerns a system that improves the robustness of automatic heartbeat detection and classification under conditions of low signal quality.

### PRIOR ART

One of the main challenges in automatic analysis of long-term Holter recordings is ensuring robustness of the automatic algorithms to low-quality ECG signals. Even after preprocessing and filtering, not all noise and artifacts can be eliminated. Low-quality signals may appear as short artifacts or as long segments of distorted, noisy signal. Artifacts, similarly to ectopic heartbeats, manifest as outlier observations and may be falsely classified as heartbeats or ectopics. Long low-quality segments distort characteristic ECG waves, which in turn causes errors in the detection and classification of heartbeats and arrhythmias. A part of the automatic analysis pipeline that is particularly sensitive to signal quality is heartbeat detection and classification. Its performance has a direct impact on the accuracy of multiple diagnostic results generated from Holter recordings, including heart rate estimation, Poincaré plot and heart rate variability (HRV) analysis, identification of the fastest and slowest arrhythmia episodes, pause detection, detection of ectopic beats, calculation of ventricular and supraventricular beat counts.

Errors in these parameters are mainly caused by falsely detected, excessive, or missing R-peaks. Such mistakes can be mitigated through appropriate channel management and self-correcting mechanisms for sensitive R-peak detection.

### BRIEF DESCRIPTION OF THE INVENTION

The first aspect of invention is a method for automatic analysis of electrocardiographic, ECG, recordings comprising the steps of:
a. Receiving an ECG recording with a plurality of ECG channels.
b. Filtering and resampling the ECG recording to a uniform frequency.
c. Assessing signal quality for each of the ECG channels and determining low-quality probabilities for short time segments.
d. Excluding ECG channels and/or segments of the plurality of ECG channels with signal quality lower than predetermined threshold.
e. Detecting R-peak candidates.
f. Verifying and classifying R-peaks.

Preferably assessing signal quality of the ECG channels and determining low-quality probabilities for short time segments of step c. are performed by a neural network preferably based on the ResNet architecture.

Preferably in the step c. the channel management module excludes a channel from R-peak detection within a time window lasting several hours if the median low-quality probability of that channel in said window exceeds a predefined threshold, and wherein a similar rule applies to channel selection for QRS classification, the evaluation window in this case being much shorter, typically lasting several tens of seconds.

Preferably the evaluation window for R-peak detection lasts several hours, for QRS classification lasts several tens of seconds.

Preferably in the step c. the QRS classifier module receives, as input, a seven-second ECG signal segment from a single channel comprising six seconds preceding and one second following a target QRS, together with ten heart rate values estimated for five preceding and five subsequent R-peak candidates.

Preferably in the step c. the multi-channel scenario the QRS classifier processes all channels recommended by the channel management module

Preferably the final classification output is obtained by averaging logarithmic responses from all processed channels.

Preferably detecting R-peak candidates of step e. are performed using a modified Pan-Tompkins algorithm adapted for multi-channel operation with averaged detection features.

Preferably verifying and classifying R-peaks from step f. is performed using a deep neural network trained to distinguish heartbeat classes.

Another aspect of the invention is a system for automatic analysis of electrocardiographic, ECG, recordings. The system comprises a low-quality prediction module configured to assess signal quality of each ECG channel by means of a deep neural network based on a ResNet architecture. The network is trained on manually annotated ECG data and outputs probabilities of low-quality occurrence for short time windows. It comprises a channel management module configured to determine, based on the probabilities provided by the low-quality prediction module, which channels or time fragments are suitable for further processing and which are to be excluded from analysis. An R-peak detection module is configured to detect candidate R-peaks using a modified Pan-Tompkins algorithm adapted for multi-channel operation, wherein detection features are calculated separately for each available channel and subsequently averaged. A QRS classifier module is configured to verify R-peak candidates and classify heartbeats, the module comprising a deep neural network based on a ResNet architecture trained on annotated ECG data and including an additional *nonQRS* class representing falsely detected R-peaks. The modules are functionally connected to cooperate in a processing pipeline to provide robust heartbeat detection and classification under varying signal quality conditions.

Yet another aspect of the invention is a computer program product comprising instructions which, when the program is executed by a computer, cause the computer to carry out the steps of the method as described previously.

Lastly a computer-readable data carrier having stored thereon the computer program product is provided.

By combining multi-channel quality prediction, adaptive channel selection, and deep-learning-based correction of R-peak detection, the described system provides a robust method for accurate heartbeat detection and classification in noisy Holter recordings. The system reduces false R-peak detections, minimizes missed beats, and improves downstream analytical results such as heart rate variability, arrhythmia identification, and ectopic beat counts. As a result, it enhances the diagnostic reliability of long-term ECG analysis under real-world conditions.

### BRIEF DESCRIPTION OF THE DRAWINGS

The invention is shown on figures, in which:
Fig. 1 illustrates the first step of signal processing - low quality prediction.
Fig. 2 illustrates the second step of signal processing - channels management.
Fig. 3 illustrates the third step of signal processing - low quality detection.
Fig. 4 illustrates the fourth step of signal processing - R-peaks detection.
Fig. 5 illustrates the fifth step of signal processing - QRS classification with R detection correction.
Fig. 6 shows examples of detection feature calculation in multichannel R detection, showing two channels excluded due to low quality contributing to the averaged detection feature.
Fig. 7 shows examples of detection feature calculation in multichannel R detection, showing all channels contributing to the averaged detection feature.
Fig. 8 shows examples of heartbeat classes (N, V, Sv) and classifier inputs: 7-second ECG segments and 10 heart rate values.

### DETAILED DESCRIPTION OF THE INVENTION

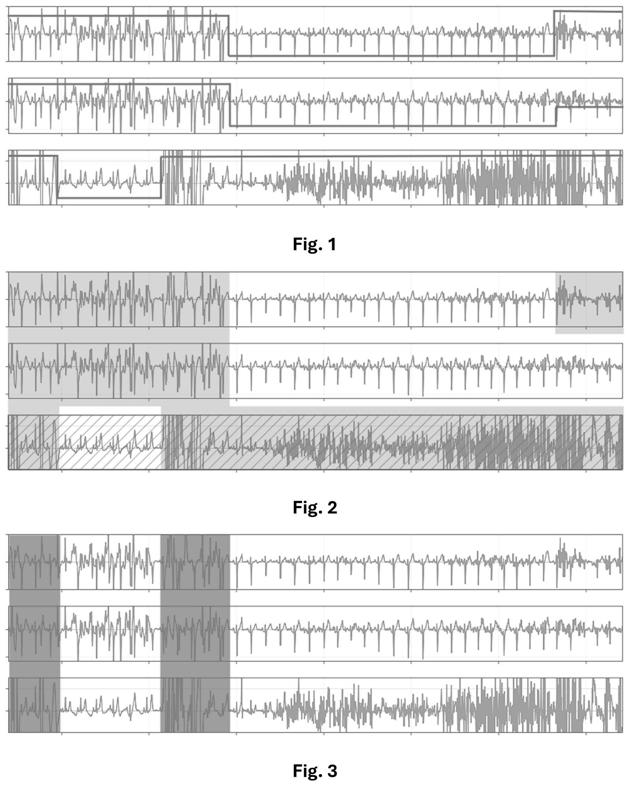

The invention concerns a method, system, and computer program for the automatic analysis of electrocardiographic (ECG) recordings, designed to provide reliable heartbeat detection and classification under varying signal quality conditions. The method operates on ECG recordings comprising multiple channels and includes several processing stages executed by interconnected modules forming an analytical pipeline. The process begins with receiving a multichannel ECG recording, which is filtered and resampled to a uniform frequency. The first stage of analysis is performed by a Low-Quality Prediction Module that automatically evaluates the signal quality of each ECG channel as seen on Fig. 1. This module employs a deep neural network based on the ResNet architecture, trained on a large manually annotated database containing examples of low-quality and artifact segments. The network analyses short time windows of three seconds and outputs probabilities of low-quality occurrence for each channel. Both short artifacts and longer noisy segments are detected. A signal fragment is designated as low-quality if the minimal probability of good quality among all channels falls below a predefined threshold, on fig.1 such probability of low quality is indicated with step line along the ECG signal.

The next stage is executed by the Channel Management Module, which determines which ECG channels and corresponding time fragments are suitable for further processing (Fig. 2). In the embodiment shown on Fig. 2 the light grey colour indicates channels mask to be excluded from QRS classification, additionally the channels which are crossed indicated the channels mask to exclude from R detection. This module is rule-based and relies on the low-quality probabilities produced by the preceding module. For R-peak detection, the median probability of low-quality signal is calculated for each channel within predefined time windows extending over several hours. If the median probability within a given window exceeds a predefined threshold, typically 0.5, that channel is excluded from R-peak detection during that period. A similar rule governs the selection of channels for QRS classification, but in this case, the evaluation windows are considerably shorter, lasting several tens of seconds, typically between 20 and 60 seconds. The result of this module is a configuration of available channels that are designated for use in subsequent analysis stages, ensuring that the system adapts to variations in signal quality. When the probability of poor signal quality remains high over consecutive windows, the detected low-quality fragments are merged into longer intervals, when the probability of poor signal quality remains high for extended duration (Fig. 3). In the embodiment as shown on Fig. 3, 4 and 5 the segments classified as low quality are indicated with dark grey colour.

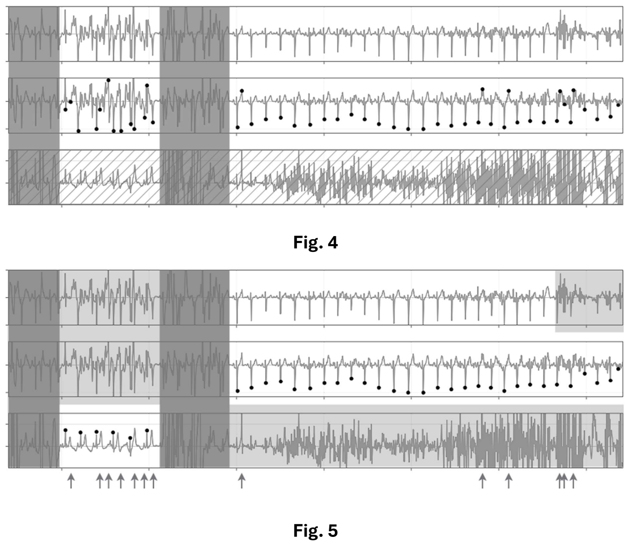

Following channel management, R-peak detection is performed by the R-Peak Detection Module (Fig. 4). This module uses a modified Pan-Tompkins algorithm adapted for multichannel operation. In the first step, a detection feature is computed separately for each available channel. The detection features from all channels recommended by the Channel Management Module are then averaged to produce a unified detection signal. The parameters of the detection function are adjusted to favor high sensitivity in detecting R-peaks, even at the expense of generating an increased number of false positives. This design choice ensures that all potential R-peak candidates are identified and passed on to the subsequent classification stage for verification, thereby reducing the risk of missed detections. The detected R-peaks candidates are marked on Fig. 4 as dots.

Verification and classification of R-peaks are performed by the QRS Classifier Module (Fig. 5), which serves both as a correction mechanism for R-peak detection and as a classifier of heartbeats into distinct categories. This module incorporates a deep neural network based on the ResNet architecture, trained on a large manually annotated database of ECG recordings containing heartbeat positions and classes. The classifier distinguishes three main heartbeat types: normal (N), ventricular (V), and supraventricular (SV). To enhance the robustness of detection and to enable correction of falsely detected R-peaks, an additional class called "nonQRS" is introduced during training. In the training process, the R-peak candidates generated by the R-Peak Detection Module are compared with reference manual annotations, and any candidate that does not correspond to a true heartbeat is labeled as "nonQRS", on the Fig. 5 such R-peaks candidates which are deleted by QRS classifier are marked with arrow. This approach allows the classifier to learn to identify and eliminate false positives regardless of variations in the preceding detection stage.

For each analyzed instance, the QRS classifier receives as input a seven-second ECG segment from a single channel, consisting of six seconds preceding and one second following the target QRS, as well as ten heart rate values estimated for the five preceding and five subsequent R-peak candidates. In multichannel scenarios, the classifier processes all channels recommended by the Channel Management Module. The final classification result is obtained by averaging logarithmic outputs from all processed channels, ensuring adaptive weighting of the most reliable signals and improving classification accuracy in the presence of variable or degraded signal conditions.

The method for automatic analysis of Holter recordings thus includes the steps of receiving a multichannel ECG recording, filtering and resampling it to a uniform frequency, assessing the signal quality for each channel and determining low-quality probabilities for short time segments using a neural network based on the ResNet architecture, excluding ECG channels or segments with signal quality below a predetermined threshold, detecting R-peak candidates using a modified Pan-Tompkins algorithm adapted for multichannel operation with averaged detection features, and verifying and classifying R-peaks using a deep neural network trained to distinguish heartbeat classes, including a "nonQRS" class. In the multichannel configuration, the classifier processes all channels selected by the Channel Management Module, and the final classification output is obtained by averaging the logarithmic responses from all processed channels.

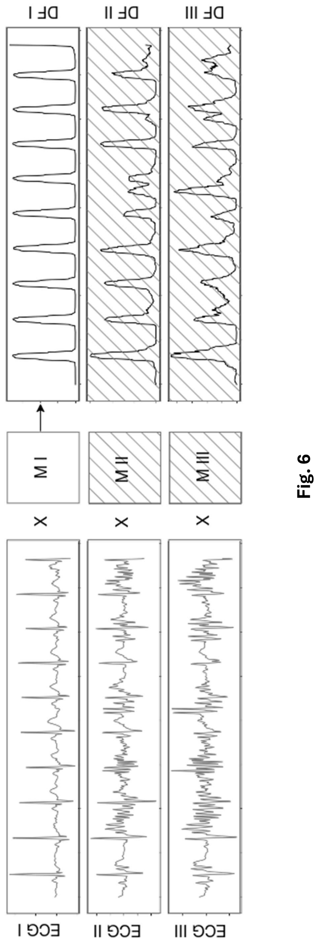

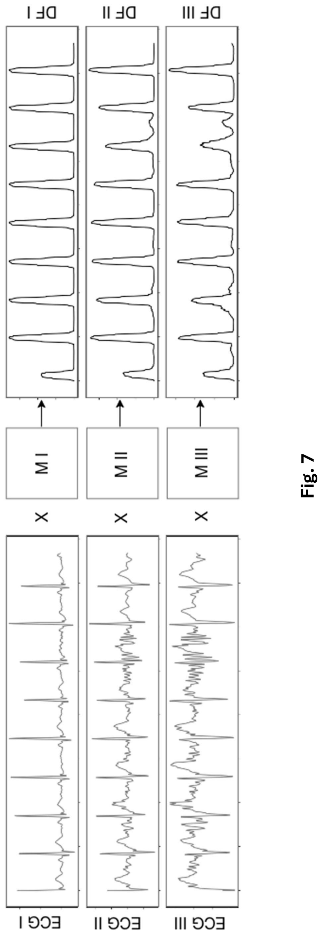

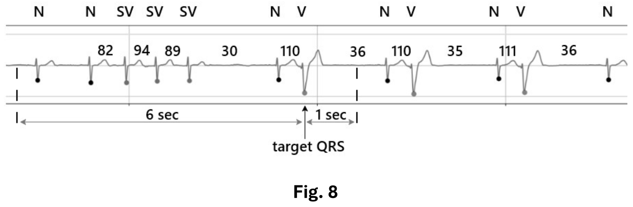

The system implementing this method comprises a Low-Quality Prediction Module configured to assess the signal quality of each ECG channel by means of a ResNet-based neural network trained on manually annotated data and producing probabilities of low-quality occurrence for short overlapping time windows; a Channel Management Module configured to determine, based on these probabilities, which channels or fragments are suitable for further processing and which should be excluded. As shown on Fig. 6 only the channel ECG I has been used for further step of R detection, wherein ECG II and ECG III have been excluded using masks M I, M II and M III. The final feature averaged detection feature is only based on first channel ECG I. As shown on Fig. 7, all three channels ECG I, II and III have been determined to have sufficient quality and therefore all detection feature DF I, DF II and DF III contribute to the averaged detection feature. An R-Peak Detection Module configured to detect candidate R-peaks using a modified Pan-Tompkins algorithm adapted for multichannel operation, wherein detection features are computed separately for each channel and subsequently averaged; and a QRS Classifier Module configured to verify R-peak candidates and classify heartbeats using a ResNet-based neural network trained on annotated ECG data and including an additional "nonQRS" class. These modules are functionally connected to cooperate in a sequential processing pipeline that ensures robust heartbeat detection and classification even when the signal quality varies significantly between channels or over time. On Fig. 8 there are presented examples of heartbeat classes N - normal, SV - supraventricular, V - ventricular as well as classifier inputs: 7-second ECG segments and 10 heart rate values.

Additionally, a computer program product and a computer-readable data carrier are provided, containing instructions that, when executed by a computer, perform the steps of the described method.

## Claims

1. A method for automatic analysis of electrocardiographic, ECG, recordings comprising the steps of:
a. receiving an ECG recording with a plurality of ECG channels,
b. filtering and resampling the ECG recording to a uniform frequency;
c. assessing signal quality for each of the ECG channels and determining low-quality probabilities for short time segments;
d. excluding ECG channels and/or segments of the plurality of ECG channels with signal quality lower than predetermined threshold;
e. detecting R-peak candidates; and
f. verifying and classifying R-peaks.

2. Method according to claim 1, wherein assessing signal quality for the ECG channels and determining low-quality probabilities for short time segments of step c. are performed by a neural network preferably based on the ResNet architecture.

3. Method according to claim 1 or 2, wherein in the step c. the channel management module excludes a channel from R-peak detection within a time window if the median low-quality probability of that channel in said window exceeds a predefined threshold, and wherein a similar rule applies to channel selection for QRS classification, the evaluation window in this case being much shorter, typically lasting several tens of seconds.

4. Method according to claim 3, wherein the evaluation window for R-peak detection lasts several hours, for QRS classification lasts several tens of seconds.

5. Method according to any of the preceding claims 1-4, wherein in the step c. the QRS classifier module receives, as input, a seven-second ECG signal segment from a single channel comprising six seconds preceding and one second following a target QRS, together with ten heart rate values estimated for five preceding and five subsequent R-peak candidates.

6. Method according to any of the preceding claims 1-5, wherein in the step c. the multi-channel scenario the QRS classifier processes all channels recommended by the channel management module

7. Method according to any of the preceding claims 1-6, wherein the final classification output is obtained by averaging logarithmic responses from all processed channels.

8. Method according to any of the preceding claims 1-7, wherein detecting R-peak candidates of step e. are performed using a modified Pan-Tompkins algorithm adapted for multi-channel operation with averaged detection features.

9. Method according to any of the preceding claims 1-8, wherein verifying and classifying R-peaks from step f. is performed using a deep neural network trained to distinguish heartbeat classes.

10. A system for automatic analysis of electrocardiographic, ECG, recordings comprising:
a low-quality prediction module configured to assess signal quality of each ECG channel by means of a deep neural network based on a ResNet architecture, wherein the network is trained on manually annotated ECG data and outputs probabilities of low-quality occurrence for short time windows;
a channel management module configured to determine, based on the probabilities provided by the low-quality prediction module, which channels or time fragments are suitable for further processing and which are to be excluded from analysis;
an R-peak detection module configured to detect candidate R-peaks using a modified Pan-Tompkins algorithm adapted for multi-channel operation,
wherein detection features are calculated separately for each available channel and subsequently averaged; and
a QRS classifier module configured to verify R-peak candidates and classify heartbeats, the module comprising a deep neural network based on a ResNet architecture trained on annotated ECG data and including an additional nonQRS class representing falsely detected R-peaks,
wherein the modules are functionally connected to cooperate in a processing pipeline to provide robust heartbeat detection and classification under varying signal quality conditions.

11. A computer program product comprising instructions which, when the program is executed by a computer, cause the computer to carry out the steps of the method of claim anyone of claims 1-9.

12. A computer-readable data carrier having stored thereon the computer program product of claim 11.
